# EUROPEAN PATENT SPECIFICATION

(11) **EP 4 049 628 B1**
(45) Date of publication and mention of the grant of the patent: **27.11.2024**
(21) Application number: 20879754.8
(22) Date of filing: 15.10.2020
(51) Int. Cl.: A61F 2/36, A61F 2/30, A61D 1/00

(54) **ANIMAL FEMORAL IMPLANT**
FEMURIMPLANTAT FÜR TIERE
IMPLANT FÉMORAL POUR ANIMAL

(30) Priority: 23.10.2019 KR 20190132173
(43) Date of publication of application: 31.08.2022
(73) Proprietor: Vetrust Meditech Co., Ltd., Seoul 06650 (KR)
(72) Inventor: SEO, Jeong-Woo, Seoul 05594 (KR); SIM, Bo-Kyun, Seoul 02623 (KR)
(74) Representative: Witte, Weller & Partner Patentanwälte mbB
(86) International application number: PCT/KR2020/014068
(87) International publication number: WO 2021/080243

(56) References cited:
- CN-A- 105 559 947
- CN-U- 203 244 471
- US-A1- 2006 190 092
- US-A1- 2006 241 776
- US-A1- 2006 241 776
- US-A1- 2011 144 762
- US-A1- 2017 056 184
- US-A1- 2019 224 015
- US-B2- 9 730 798

## Description

### Technical Field

The present invention relates to an animal femoral implant and, more specifically, to an animal femoral implant, which may enable artificial hip joint replacement for animals, may enable the implant to be firmly fixed to the animal femur by spontaneous bone growth of the animal, thereby preventing complications such as aseptic dissociation and bone resorption around the cement, which may occur when using bone cement, and may cause a porous part, which has relatively low strength due to a plurality of pores formed therein, to be protected by a frame part, which has relatively high strength due to a solid face formed therein, thereby preventing damage to the porous part in which the edge thereof is broken or bent by friction with the bone or by an external force in the process of inserting the femur implant into the animal femur and eliminating a problem in that porous particles that may be generated when the porous part is damaged penetrate into blood vessels and the like to cause various inflammatory reactions.

### Background Art

The hip joint is a portion where the outer acetabular fossa of the pelvis and the femoral head of the femur meet, and is configured in the form of a ball- and-socket joint to enable joint movement in various directions.

The acetabular fossa and the femoral head are covered with articular cartilage, and the labrum covers the femoral head on the outside of the acetabular fossa together with the acetabular fossa, and the articular capsule surrounds both the acetabular fossa and the femoral neck on the outside thereof.

The femur constituting the hip joint is divided into the ball-shaped femoral head, the femoral neck that is tapered from the femoral head and connected to the intertrochanter, and the intertrochanter, which is a part for connecting the greater trochanter and the lesser trochanter, having various muscles attached thereto, depending on the region.

If arthritis, fractures, or other diseases occur in the hip joint to cause extreme pain, and if the symptoms are not improved even with physical therapy or medication, artificial hip joint replacement, which is surgery to replace the problematic hip joint with an artificial hip joint, is considered.

The total artificial hip joint replacement indicates surgery to replace the entire hip joint with an artificial joint after removing the damaged bone and articular cartilage.

The artificial hip joint replacement is performed on the femur by removing the femoral head and inserting a metal femoral stem into the femoral marrow cavity to be fixed, and a metal or ceramic-type ball is inserted into the top end of the femoral stem to replace the removed femoral head.

The artificial hip joint replacement is performed on the pelvis by removing the damaged acetabular fossa articular cartilage, inserting an acetabular fossa cup, which is a metal socket, into the acetabular fossa to be fixed using metal screws or cement, and inserting a plastic, ceramic, or metal liner into the acetabular fossa cup such that the ball and the socket come into smooth contact with each other.

In addition to the above-described total artificial hip joint replacement, artificial hip joint replacement may include the artificial joint hemiarthroplasty in which surgery is performed only on the femur in the case where there is damage or disease in the femoral head articular cartilage but there is no problem on the acetabular fossa and surface replacement in which the socket on the acetabular fossa is similar to the total replacement and in which an artificial joint implant is inserted to cover the femoral head, instead of removing the femur head.

US 2017/056184 A1 discloses a femoral stem prosthesis.

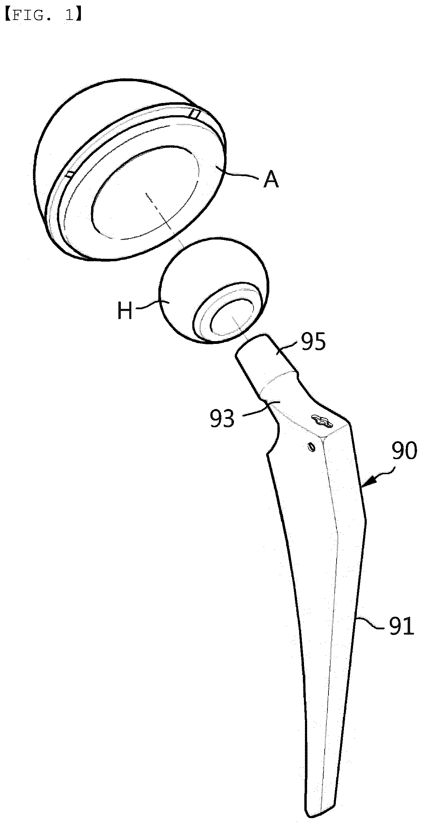

FIG. 1 is a view illustrating a femur implant 90 used in the conventional artificial hip joint replacement, which is disclosed in Korean Patent Application Laid-Open No. 10-2005-0112919 (01 December, 2005). The conventional femur implant 90 includes a body part 91, a neck part 93, and a coupling part 95.

The body part 91 indicates a configuration that is inserted and fixed into the marrow cavity of the femur, and the neck part 93 indicates a configuration that is formed to extend in the proximal direction from the body part 91 to connect the coupling part 95 and the body part 91, and the coupling part 95 indicates a configuration that is formed in the truncated-cone shape at the proximal end of the femur implant 90 such that an artificial femoral head H in contact with an acetabular fossa cup A is fitted and assembled to the same.

This femur implant 90 is fixed into the femur marrow cavity by normally using bone cement, and it was confirmed, as a result of mid- to long-term follow-up of patients who received cement-type femur implants 90, that many complications such as aseptic dissociation and severe bone resorption around the cement occurred. Therefore, active research on a cementless implant in which the femur implant 90 is fixed to the femur without using cement is underway.

Since the femur implant 90 discussed above is intended for humans, there is a limit in that it cannot be applied to animals such as dogs and cats, which have a different anatomical structure from people who walk upright. Along with the improvement of living standards, as the number of people who keep companion animals has significantly increased due to the decline of nuclear family and the like, the demand for development of medical technology to treat animals with disabilities, as well as humans, is increasing day by day, but the reality is that there is no dedicated medical equipment for animals.

When a disease similar to a human disease occurs in the animal's hip joint, there is no dedicated implant to replace the animal's hip joint, which makes it impossible to provide an appropriate procedure such as artificial hip joint replacement to the animal.

Accordingly, the related industry is demanding the introduction of new technology capable of performing artificial hip joint replacement on animals with the development of implants for animals by reflecting the anatomy of animals, thereby enabling normal gait of the treated animal and restoring the joint movement of the animal to its original state.

### Disclosure of Invention

### Technical Problem

The present invention has been devised to solve the above problems.

An aspect of the present invention is to provide an animal femoral implant to enable artificial hip joint replacement for animals.

Another aspect of the present invention is to configure a body part, a neck part extending from the proximal portion of the body part, and a coupling part extending from the proximal portion of the neck part, wherein the body part is inserted and fixed into the animal femur, wherein interference within a certain range is prevented by the neck part so as to restore the anatomical joint movement of the animal, and wherein the coupling part is provided such that an artificial femoral head is coupled to the coupling part.

Another aspect of the present invention is to configure a bone growth part in the proximal portion of the body part, so that the body part can be firmly fixed to the animal femur by spontaneous bone growth of the animal even without using bone cement.

Another aspect of the present invention is to configure a stem part in the distal portion of the body part so as to facilitate insertion of the body part into the femur marrow cavity of an animal and stably fix the body part into the femur by the inserted stem part.

Another aspect of the present invention is to configure a bone growth part to extend in the distal direction while forming a step against the distal end of the neck part to increase the surface area of the bone growth part, thereby promoting bone growth, and preventing the range of joint movement of the implant from being limited, compared to the range of anatomical joint movement, due to the interference between the neck part and the acetabular fossa cup.

Another aspect of the present invention is to configure a porous part having a plurality of pores formed in the bone growth part so that the implant is fixed to the femur by bone growth and so that bone cement is not used, thereby preventing complications such as aseptic dissociation and bone resorption around the cement that may occur when using bone cement.

Another aspect of the present invention is to configure a frame part in the bone growth part, so that the porous part having relatively weak strength due to a plurality of pores formed therein can be protected by the frame part.

Another aspect of the present invention is to form a solid face such that a relatively strong frame part is formed along the edge of the bone growth part, thereby preventing damage to the porous part, which has weak strength, in which the edge thereof is broken or bent by friction with the bone or by an external force in the process of inserting the femur implant into the femur of an animal, and eliminating a problem in that porous particles that may be generated when the porous part is damaged penetrate into blood vessels and the like to cause various inflammatory reactions.

Another aspect of the present invention is to manufacture a porous part having a plurality of pores and a frame part forming a solid face by 3D printing, thereby facilitating the fabrication of femur implants for animals and providing implants customized for each animal.

Another aspect of the present invention is to configure the porous part and the frame part, which are adjacent, to have the same level so that the relatively weak porous part can be protected by the frame part and so that the porous part comes into close contact with the intraosseous wall when the body part is inserted into the marrow cavity of an animal, thereby promoting bone growth.

Another aspect of the present invention is to configure a stem part to extend in the distal direction while being reduced from the distal end of the bone growth part to form a step, thereby facilitating insertion of an animal femoral implant into the femur marrow cavity of an animal.

As the size of animal increases, for example, small animals, medium animals, and large animals, the size of an artificial femoral head coupled to the animal femoral implant increases, and the size of the coupling part also increases. In the case where the coupling part is relatively large compared to the neck part, the neck part becomes relatively thin and fails to withstand the concentrated stress to be easily damaged. Thus, another aspect of the present invention is to configure an animal femoral implant in which the larger the coupling part, the larger the neck part, and the smaller the coupling part, the smaller the neck part, thereby preventing damage to the neck part while minimizing the interference problem by the neck part.

Another aspect of the present invention is to configure a locking hole in the bone growth part such that a locking bolt engages with the locking hole, thereby preventing sinking of the animal femoral implant inserted into the animal femur.

Another aspect of the present invention is to configure a locking hole on the outer surface of a bone growth part so that a locking bolt may be inserted from the outside to the inside of the animal femur, thereby ensuring the field of view of a surgeon and the convenience of surgery in the artificial hip joint replacement surgery.

Another aspect of the present invention is to configure a locking hole such that the hole center axis thereof is perpendicular to the stem axis, thereby facilitating the surgeon to fasten the locking bolt to the locking hole in a surgical situation where the field of view of the surgeon is extremely limited by blood, body fluid, foreign substances, etc.

Another aspect of the present invention is to configure a locking hole such that the hole center axis thereof is inclined at a certain angle with respect to the stem axis, instead of being perpendicular thereto, thereby more effectively preventing sinking of the implant inserted into the animal femur.

### Solution to Problem

In order to solve the problems described above, the present invention is implemented by an embodiment having the following configuration.

The present invention includes a body part inserted into the femur of an animal, a coupling part to which an artificial femoral head is coupled, and a neck part connecting the body part and the coupling part.

According to the present invention, the body part includes a bone growth part formed in the proximal portion to promote bone growth and a stem part formed in the distal portion to facilitate insertion into the femur.

According to the present invention, the bone growth part is formed to extend in the distal direction to be expanded from the distal end of the neck part while forming a step.

According to the present invention, the bone growth part includes a porous part having a plurality of pores formed therein to promote bone growth.

According to the present invention, the bone growth part includes a frame part that forms a solid face to protect the porous part.

According to the present invention, the frame part is formed along the edge of the bone growth part.

According to another embodiment of the present invention, the porous part and the frame part are manufactured by 3D printing.

According to another embodiment of the present invention, the porous part and the frame part, which are adjacent, have the same level.

According to the present invention, the stem part is formed to extend in the distal direction from the distal end of the bone growth part so as to be reduced for forming a step.

According to another embodiment of the present invention, the size of the neck part varies depending on the size of the coupling part in the animal femoral implant.

According to another embodiment of the present invention, as the size of the coupling part increases, the size of the neck part increases, and as the size of the coupling part decreases, the size of the neck part decreases in the animal femoral implant.

According to another embodiment of the present invention, the present invention includes a body part inserted into the femur of an animal, a coupling part to which an artificial femoral head is coupled, and a neck part connecting the body part and the coupling part, wherein the body part includes a bone growth part formed in the proximal portion to promote bone growth and a stem part formed in the distal portion to facilitate insertion into the femur, and wherein the bone growth part further includes a locking hole into which a locking bolt is inserted to prevent sinking.

According to another embodiment of the present invention, the locking hole is formed on the outer surface of the bone growth part.

According to another embodiment of the present invention, a hole central axis of the locking hole is perpendicular to a stem axis.

According to another embodiment of the present invention, a hole central axis of the locking hole is not perpendicular to the stem axis.

### Advantageous Effects of Invention

The present invention can obtain the following effects by the embodiment, configuration, combination, and usage relationship described, which will be described below.

The present invention has an effect of providing an animal femoral implant to enable artificial hip joint replacement for animals.

The present invention provides an effect of configuring a body part, a neck part extending from the proximal portion of the body part, and a coupling part extending from the proximal portion of the neck part, wherein the body part is inserted and fixed into the animal femur, wherein interference within a certain range is prevented by the neck part so as to restore the anatomical joint movement of the animal, and wherein the coupling part is provided such that an artificial femoral head is coupled to the coupling part.

The present invention has an effect of firmly fixing the body part to the animal femur by spontaneous bone growth of the animal even without using bone cement by configuring a bone growth part in the proximal portion of the body part.

The present invention has an effect of facilitating insertion of the body part into the femur marrow cavity of an animal and stably fixing the body part into the femur by an inserted stem part by configuring a stem part in the distal portion of the body part.

The present invention provides an effect of configuring a bone growth part to extend in the distal direction while forming a step against the distal end of the neck part to increase the surface area of the bone growth part, thereby promoting bone growth, and preventing the range of joint movement of the implant from being limited, compared to the range of anatomical joint movement, due to the interference between the neck part and the acetabular fossa cup.

The present invention has an effect of configuring a porous part having a plurality of pores formed in the bone growth part so that the implant is fixed to the femur by bone growth and so that bone cement is not used, thereby preventing complications such as aseptic dissociation and bone resorption around the cement that may occur when using bone cement.

The present invention provides an effect of configuring a frame part in the bone growth part, so that the porous part having relatively weak strength due to a plurality of pores formed therein can be protected by the frame part.

The present invention provides an effect of forming a solid face such that a relatively strong frame part is formed along the edge of the bone growth part, thereby preventing damage to the weak porous part in which the edge thereof is broken or bent by friction with the bone or by an external force in the process of inserting the femur implant into the femur of an animal, and eliminating a problem in that porous particles that may be generated when the porous part is damaged penetrate into blood vessels and the like to cause various inflammatory reactions.

The present invention has an effect of facilitating the fabrication of femur implants for animals and providing implants customized for each animal by manufacturing a porous part having a plurality of pores and a frame part forming a solid face by 3D printing.

The present invention has an effect of configuring the porous part and the frame part, which are adjacent, to have the same level so that the relatively weak porous part can be protected by the frame part and so that the porous part comes into close contact with the intraosseous wall when the body part is inserted into the marrow cavity of an animal, thereby promoting bone growth.

The present invention has an effect of configuring a stem part to extend in the distal direction while reducing from the distal end of the bone growth part to form a step, thereby facilitating insertion of an animal femoral implant into the femur marrow cavity of an animal.

As the size of animal increases such as small animals, middle animals, and large animals, the size of an artificial femoral head coupled to the animal femoral implant increases, and the size of the coupling part also increases. In the case where the coupling part is relatively large compared to the neck part, the neck part becomes relatively thin and fails to withstand the concentrated stress to be easily damaged. Thus, the present invention has an effect of configuring an animal femoral implant in which the larger the coupling part, the larger the neck part, and the smaller the coupling part, the smaller the neck part, thereby preventing damage to the neck part while minimizing the interference problem by the neck part.

The present invention has an effect of preventing sinking of the animal femoral implant inserted into the animal femur by configuring a locking hole in the bone growth part such that a locking bolt engages with the locking hole.

The present invention provides an effect of ensuring the field of view of a surgeon and the convenience of surgery in the artificial hip joint replacement surgery by configuring a locking hole on the outer surface of a bone growth part so that a locking bolt may be inserted from the outside to the inside of the animal femur.

The present invention has an effect of facilitating the surgeon to fasten the locking bolt to the locking hole in a surgical situation where the field of view of the surgeon is extremely limited by blood, body fluid, foreign substances, etc. by configuring a locking hole such that the hole center axis thereof is perpendicular to the stem axis.

The present invention has an effect of more effectively preventing sinking of the implant inserted into the animal femur by configuring a locking hole such that the hole center axis thereof is inclined at a certain angle with respect to the stem axis, instead of being perpendicular thereto.

### Brief Description of Drawings

FIG. 1 is a view illustrating a femur implant used in a conventional artificial hip joint replacement.
FIG. 2 is a perspective view of an animal femoral implant according to an embodiment of the present invention.
FIG. 3 is a view illustrating the animal femoral implant in FIG. 2 when viewed from the anterior side.
FIG. 4 is a view illustrating the animal femoral implant in FIG. 2 when viewed from the inside.
FIG. 5 is a view illustrating the animal femoral implant in FIG. 2 when viewed from a proximal portion.
FIG. 6 is a view illustrating the animal femoral implant in FIG. 2 when viewed from a distal portion.
FIG. 7 is a view showing that a porous part and a frame part, which are adjacent, have the same level.
FIG. 8 is a view showing that the size of a neck part varies depending on the size of a coupling part.
FIG. 9 is a view illustrating an animal femoral implant according to another embodiment of the present invention.
FIG. 10 is a view illustrating an animal femoral implant according to another embodiment of the present invention.
FIG. 11 is a view illustrating the usage state of the present invention.

### Best Mode for Carrying out the Invention

Hereinafter, preferred embodiments of an animal femoral implant according to the present invention will be described in detail with reference to the accompanying drawings. In the following description of the present invention, if it is determined that a detailed description of a well-known function or configuration may unnecessarily obscure the gist of the present invention, the detailed description thereof will be omitted. Unless otherwise specified, all terms in this specification have the same meaning as the terms generally understood by those of ordinary skill in the art to which the present invention belongs, and in case of conflict of meaning therewith, it will be construed as the definition used in the specification.

A femur implant 1 for animals according to the present invention is an implant that is transplanted implanted into the femur of an animal to enable artificial hip joint replacement for animals, and the entire implant 1 may be preferably manufactured by 3D printing. The femur implant 1 for animals may have a porous part such that the implant may be firmly fixed to the animal femur by spontaneous bone growth of the animal without bone cement, thereby preventing complications such as aseptic dissociation and bone resorption around the cement, which may occur when using the bone cement.

In addition, the porous part, which has relatively low strength due to a plurality of pores formed therein, may be protected by a frame part that has relatively high strength due to a solid face formed therein, thereby preventing damage to the porous part in which the edge thereof is broken or bent by friction with the bone or by an external force in the process of inserting the femur implant 1 into the animal femur and eliminating a problem in that porous particles that may be generated when the porous part is damaged penetrate into blood vessels and the like to cause various inflammatory reactions.

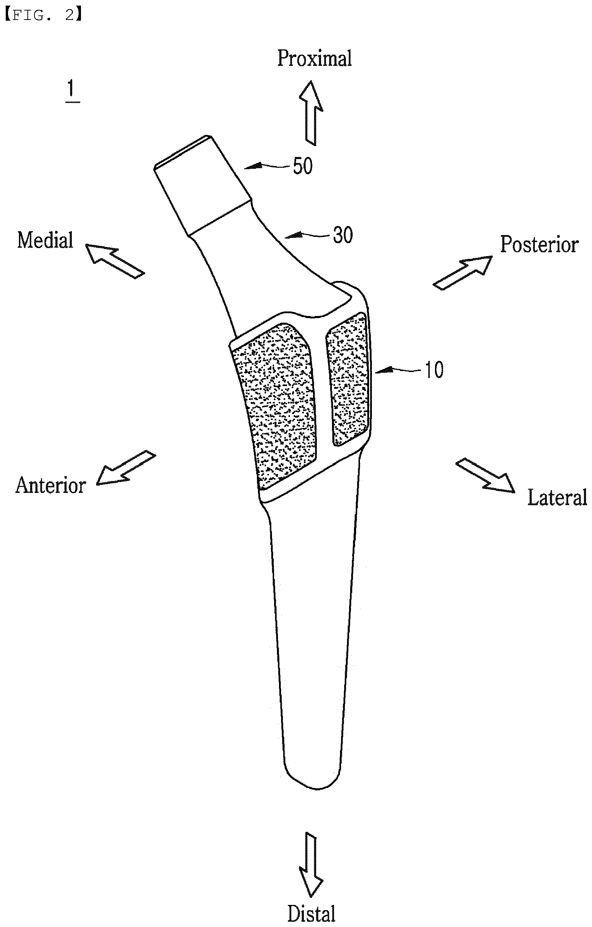

FIG. 2 is a perspective view of a femur implant 1 for animals according to an embodiment of the present invention. Referring to FIG. 2, the femur implant 1 for animals according to the present invention includes, as primary elements, a body part 10, a neck part 30, and a coupling part 50.

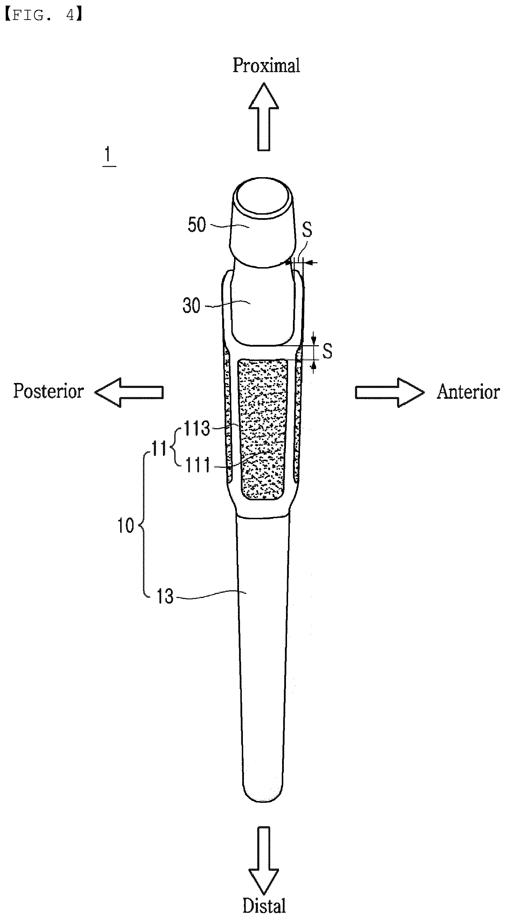

The body part 10 is a configuration to be inserted into the femur of an animal and primarily indicates the remaining portions, excluding the neck part 30 and the coupling part 50, which will be described later. The body part 10 is a part to be implanted into the femur marrow cavity of an animal, and is preferably configured to be easily inserted into the marrow cavity and to be rapidly fused with the bone after being implanted into the correct place. To this end, the body part 10 may have a tapered shape in which the cross-sectional area is reduced toward the distal portion as shown in FIGS. 2 to 4. The method of manufacturing the body part 10 is not limited to any specific concept, and, preferably, it may be manufactured by 3D printing.

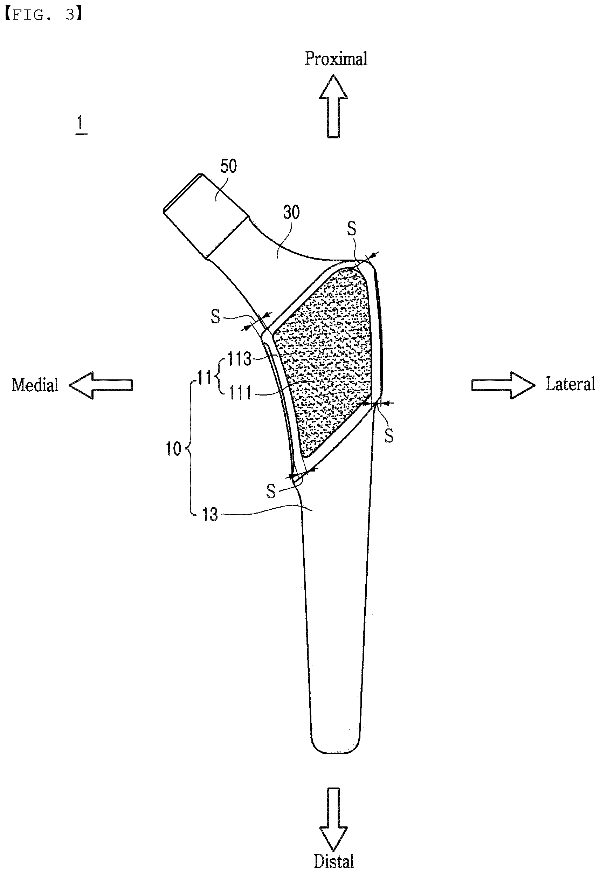

FIG. 3 is a view of the femur implant 1 for animals in FIG. 2 when viewed from the anterior side. Referring to FIG. 3, the body part 10 includes a bone growth part 11 and a stem part 13.

The bone growth part 11 is a configuration formed in the proximal portion of the body part 10 to promote bone growth, and the bone growth part 11 may be formed to extend in the distal direction while expanding from the distal end of the neck part 30, which will be described later, to form a step S. Accordingly, bone growth is promoted by increasing the surface area of the bone growth part 11, thereby preventing the range of joint movement of the implant from being limited, compared to the range of anatomical joint movement, due to interference between the neck part 30 and the acetabular fossa cup, which will be described later. Since natural bone growth is promoted through the bone growth part 11, bone cement may not be used in the process of fixing the body part 10 to the animal femur.

The bone growth part 11 includes a porous part 111 and a frame part 113.

The porous part 111 may be configured to have a plurality of pores formed to promote bone growth, enabling the femur implant 1 for animals to be fixed to the femur by bone growth so as not to use bone cement, thereby preventing complications such as aseptic dissociation and bone resorption around the cement, which may occur when using bone cement. The porous part 111 may be preferably formed by 3D printing.

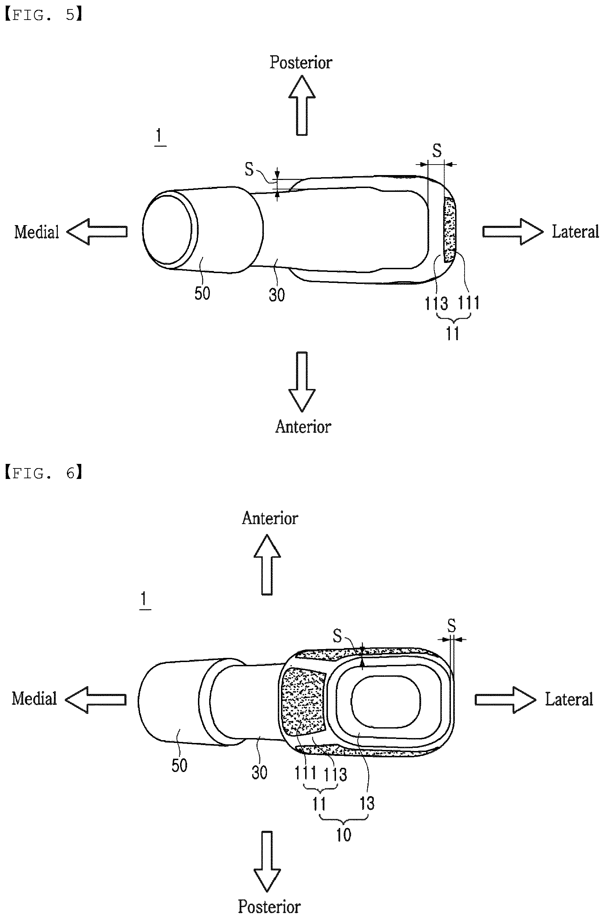

The frame part 113 is a configuration that forms a solid face and, preferably, may be formed along the edge of the bone growth part 11. That is, as shown in FIGS. 2 to 6, it is preferable that the frame part 113 is formed in all of the portions where the porous structure layers meet to form a boundary in the porous part 111. As shown in FIGS. 2 to 6, the porous part 111 may be formed on four surfaces of the body part 10, and the frame part 113 may be formed at the edge where the surfaces meet to protect the edge of the porous part 111. The frame part 113 may be configured in the bone growth part 11 so that the porous part 111, which has relatively low strength due to a plurality of pores formed therein, may be protected by the frame part 113, thereby preventing damage to the weak porous part 111 in which the edge thereof is broken or bent by friction with the bone or by an external force in the process of inserting the femur implant 1 for animals into the animal femur, and eliminating a problem in that porous particles that may be generated when the porous part 111 is damaged penetrate into blood vessels and the like to cause various inflammatory reactions.

The porous part 111 and the frame part 113 may be manufactured by 3D printing. Accordingly, the femur implant 1 for animals may be manufactured more easily, and a customized implant suitable for each animal may be provided.

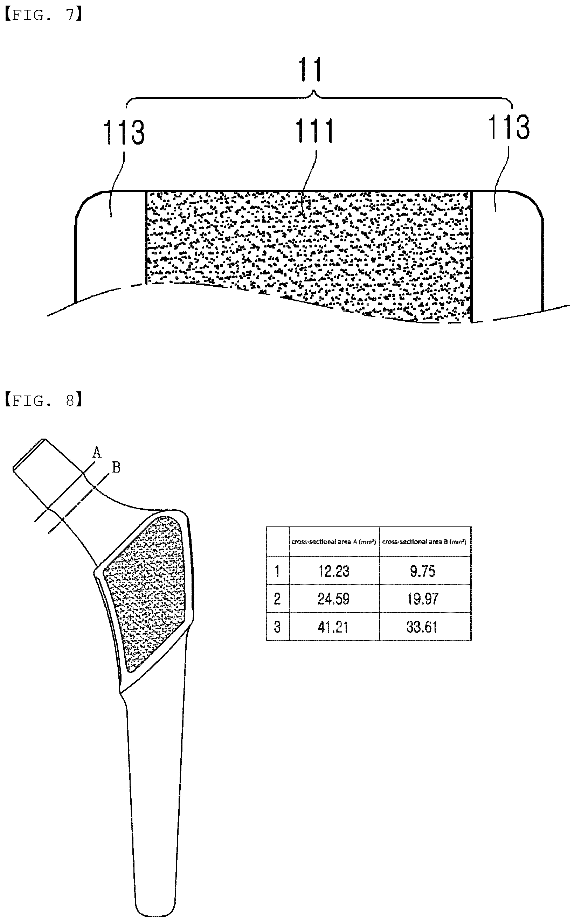

Referring to FIG. 7, the adjacent porous part 111 and frame part 113 may be configured to have the same level. The same level indicates that the portions where the porous part 111 and the frame part 113 meet have the same height, as shown in FIG. 7, while no one thereof protrudes. Through this configuration, the relatively weak porous part 111 may be protected by the frame part 113 and the porous part 111 may come into close contact with the intraosseous wall when the body part 10 is inserted into the marrow cavity of an animal, thereby promoting bone growth.

The stem part 13 is a configuration formed in the distal portion of the body part 10 to facilitate insertion of the body part 10 into the femur marrow cavity of an animal and stably fix the body part into the femur by the inserted stem part 13. The stem part 13 may be formed to extend from the distal end of the bone growth part 11 in the distal direction so as to be reduced while forming a step S. Accordingly, the femur implant 1 for animals may be easily inserted into the femur marrow cavity of an animal. As shown in FIGS. 2 to 4, the stem part 13 may be configured to have a solid surface formed thereon to reduce frictional force by a smooth surface and increase physical strength. The stem part 13 may also be manufactured by 3D printing.

The neck part 30 is a configuration for connecting the body part 10 and a coupling part 50, which will be described later, and interference within a certain range may be prevented by the neck part 30 to restore the anatomical joint movement of the animal. The femur implant 1 for animals may be configured to vary in the size of the neck part 30 depending on the size of the coupling part 50 to be described later.

Referring to FIG. 8, for example, if the cross-sectional area of the point A in the coupling part 50 is 12.23 mm², the cross-sectional area of the point B in the neck part 30 may be 9.75 mm², and if the cross-sectional area of the point A in the coupling part 50 is 24.59 mm², the cross-sectional area of the point B in the neck part 30 may be 19.97 mm², and if the cross-sectional area of the point A in the coupling part 50 is 41.21 mm², the cross-sectional area of the point B in the neck part 30 may be 33.61 mm².

That is, the femur implant 1 for animals may be configured such that as the size of the coupling part 50 to be described later increases, the size of the neck part 30 increases and such that as the size of the coupling part 50 to be described later decreases, the size of the neck part 30 decreases.

Unlike humans whose body sizes are similar between normal adults, animals are significantly different in their body sizes among small animals, middle animals, and large animals. As the size of animal increases, for example, small animals, middle animals, and large animals, the size of an artificial femoral head coupled to the femur implant 1 for animals increases, and the size of the coupling part 50, which will be described later, also increases. In the case where the coupling part 50 is relatively large compared to the neck part 30, the neck part 30 becomes relatively thin and fails to withstand the concentrated stress to be easily damaged.

Thus, in the present invention, a femur implant 1 for animals may be configured such that the larger the coupling part 50, the larger the neck part 30 and such that the smaller the coupling part 50, the smaller the neck part 30, thereby preventing damage to the neck part 30 while minimizing the interference problem by the neck part 30.

The coupling part 50 is a configuration to which the artificial femoral head is coupled, and may be formed to extend from the proximal portion of the neck part 30. Although the coupling part 50 is not limited to any specific shape, it may preferably be configured to have a truncated-cone shape. The coupling part 50 may be machined to satisfy the coupling condition with the artificial femoral head. That is, after the entire implant 1 is manufactured by 3D printing, only the coupling part 50 may be separately machined.

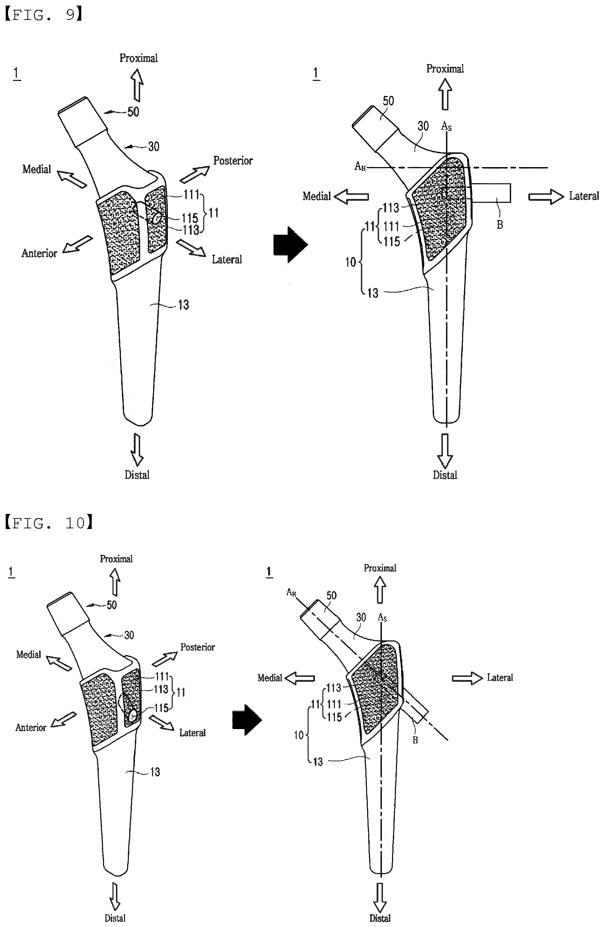

FIG. 9 is a view of a femur implant 1 for animals according to another embodiment of the present invention, in which a locking hole 115 is added to the bone growth part 11, so the following description will focus on the newly added locking hole 115 in order to avoid duplicate descriptions.

The locking hole 115 is a configuration to which a locking bolt is inserted to prevent sinking of the implant, and may be formed on the outer surface of the bone growth part 11 as shown in FIG.9. Preferably, the locking hole 115 may be machined. By configuring the locking hole 115 in the bone growth part 11 such that a locking bolt may engage with the locking hole 115, it is possible to prevent sinking of the animal femur implant 1 inserted into the animal femur.

In addition, among the bone growth part 11, the locking hole 115 may be formed on the outer surface of the bone growth part 11 such that a locking bolt may be inserted from the outside to the inside of the animal femur, thereby ensuring the field of view of a surgeon and the convenience of surgery in the artificial hip joint replacement surgery.

The locking hole 115 may be configured such that a hole center axis AH is perpendicular to a stem axis AS. Accordingly, the surgeon may more easily fasten the locking bolt to the locking hole 115 in a surgical situation in which the field of view of a surgeon is extremely limited by blood, body fluid, foreign substances, or the like.

FIG. 10 is a view of a femur implant 1 for animals according to another embodiment of the present invention, in which the locking hole 115 is formed on the bone growth part 11 to be inclined. That is, unlike that shown in FIG. 9, the locking hole 115 is configured such that the hole center axis is not perpendicular to the stem axis. As shown in FIG. 10, the locking hole 115 may be configured such that the hole center axis A_{H} is inclined at a certain angle with respect to the stem axis A_{S}, instead of being perpendicular thereto, it is possible to more effectively prevent the sinking of the implant inserted into the animal femur. Although the inclination of the locking hole 115 is directed in the distal direction in FIG. 10, the inclination direction of the locking hole 115 may be configured to be directed in the proximal direction opposite the same.

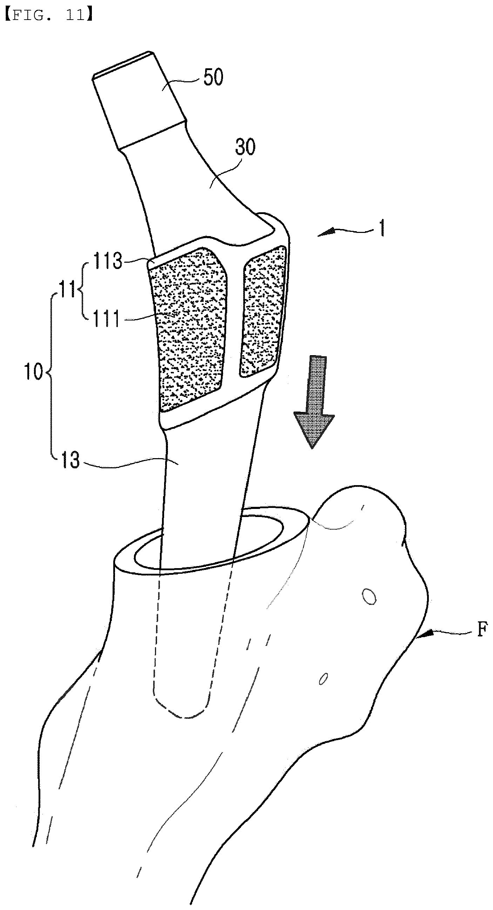

FIG. 11 is a view showing the usage state of the present invention. Referring to FIG. 11, a femur implant 1 for animals according to the present invention may be regarded as a prosthesis inserted into the marrow cavity of the animal femur F during artificial hip joint replacement surgery for animals, as shown in FIG. 11. The femur implant 1 for animals enables artificial hip joint replacement for animals, and, as described above, the femur implant 1 for animals does not require bone cement by configuring the porous part 111, thereby preventing various problems that may be caused by the use of bone cement.

Above all, the porous part 111 may form a solid face so as to be protected by a relatively strong frame part 113, thereby preventing damage to the porous part 111 in which the edge thereof is broken or bent by friction with the bone or by an external force in the process of inserting the femur implant 1 for animals into the animal femur. Porous particles, which are fine particles separated from the porous part 111, may be generated when the porous part is damaged and penetrate into blood vessels and the like to cause various inflammatory reactions. However, the present invention fundamentally prevents damage to the porous part 111 through the configuration of the frame part 113, and thus may also prevent problems caused by the porous particles.

The above detailed description exemplifies the present invention. In addition, the above description shows preferred embodiments of the present invention. The disclosed embodiment is intended to describe the best mode for implementing the technical idea of the present invention. Accordingly, the detailed description of the present invention is not intended to limit the present invention to the disclosed embodiments but is limited to the appended claims. Also, the appended claims should be construed as encompassing other embodiments.

## Claims

1. An animal femoral implant (1) comprising
a body part (10) insertable into the femur of an animal, the body part comprising a bone growth part (11) formed in the proximal portion to promote bone growth and a stem part (13) formed in the distal portion to facilitate insertion into the femur, the bone growth part (11) comprising a porous part (111) having a plurality of pores formed therein to promote bone growth,
a coupling part (50) to which an artificial femoral head is coupled, and
a neck part (30) connecting the body part and the coupling part, **characterized in that**
the bone growth part (11) is formed to extend in the distal direction while being expanded from the distal end of the neck part (30) to form a step (S),
that the stem part (13) is formed to extend in the distal direction while being reduced from the distal end of the bone growth part (11) to form a step,
that the bone growth part (11) further comprises a frame part (113) that forms a solid face to protect the porous part (111), and
that the frame part (113) is formed along the edge of the bone growth part (11).

2. The animal femoral implant of claim 1, wherein the porous part and the frame part are manufactured by 3D printing.

3. The animal femoral implant of claim 1, wherein the porous part and the frame part, which are adjacent, have the same height.

4. The animal femoral implant of claim 1, wherein a size of the neck part varies depending on a size of the coupling part.

5. The animal femoral implant of claim 4, wherein as the size of the coupling part increases, the size of the neck part increases, and wherein as the size of the coupling part decreases, the size of the neck part decreases.

6. The animal femoral implant of claim 1,
wherein the bone growth part (11) further comprises a locking hole (115) into which a locking bolt is insertable to prevent sinking.

7. The animal femoral implant of claim 6, wherein the locking hole (115) is formed on the outer surface of the bone growth part (11).

8. The animal femoral implant of claim 7, wherein a hole central axis of the locking hole (115) is perpendicular to a stem axis.

9. The animal femoral implant of claim 7, wherein a hole central axis of the locking hole (115) is not perpendicular to the stem axis.

## Patentansprüche

1. Femurimplantat (1) für Tiere, aufweisend
einen Körperteil (10), der in den Femur eines Tieres einführbar ist, wobei der Körperteil einen Knochenwachstumsteil (11), der in dem proximalen Abschnitt ausgebildet ist, um das Knochenwachstum zu fördern, und einen Schaftteil (13), der in dem distalen Abschnitt ausgebildet ist, um das Einführen in den Femur zu erleichtern, wobei der Knochenwachstumsteil (11) einen porösen Teil (111) mit einer Vielzahl von darin ausgebildeten Poren aufweist, um das Knochenwachstum zu fördern,
ein Kopplungsteil (50), an das ein künstlicher Femurkopf gekoppelt ist, und
ein Halsteil (30), das das Körperteil und das Kopplungsteil verbindet, **dadurch gekennzeichnet, dass**
das Knochenwachstumsteil (11) so geformt ist, dass es sich in distaler Richtung erstreckt, während es vom distalen Ende des Halsteils (30) aus erweitert ist, um eine Stufe (S) zu bilden,
dass der Schaftteil (13) so geformt ist, dass er sich in distaler Richtung erstreckt, während er vom distalen Ende des Knochenwachstumsteils (11) aus reduziert ist, um eine Stufe zu bilden,
dass der Knochenwachstumsteil (11) ferner einen Rahmenteil (113) aufweist, der eine feste Fläche zum Schutz des porösen Teils (111) bildet, und
dass das Rahmenteil (113) entlang der Kante des Knochenwachstumsteils (11) ausgebildet ist.

2. Femurimplantat für Tiere nach Anspruch 1, wobei das poröse Teil und das Rahmenteil durch 3D-Druck hergestellt sind.

3. Femurimplantat für Tiere nach Anspruch 1, wobei das poröse Teil und das Rahmenteil, die benachbart sind, die gleiche Höhe aufweisen.

4. Femurimplantat für Tiere nach Anspruch 1, wobei eine Größe des Halsteils in Abhängigkeit von der Größe des Kupplungsteils variiert.

5. Femurimplantat für Tiere nach Anspruch 4, wobei mit zunehmender Größe des Kopplungsteils die Größe des Halsteils zunimmt und wobei mit abnehmender Größe des Kopplungsteils die Größe des Halsteils abnimmt.

6. Femurimplantat für Tiere nach Anspruch 1,
wobei der Knochenwachstumsteil (11) ferner ein Verriegelungsloch (115) aufweist, in das ein Verriegelungsbolzen eingesetzt werden kann, um ein Einsinken zu verhindern.

7. Femurimplantat für Tiere nach Anspruch 6, wobei das Verriegelungsloch (115) an der Außenfläche des Knochenwachstumsteils (11) ausgebildet ist.

8. Femurimplantat für Tiere nach Anspruch 7, wobei eine Lochmittelachse des Verriegelungslochs (115) senkrecht zu einer Schaftachse ist.

9. Femurimplantat für Tiere nach Anspruch 7, wobei eine Lochmittelachse des Verriegelungslochs (115) nicht senkrecht zur Schaftachse ist.

## Revendications

1. Implant fémoral pour animaux (1) comprenant
une partie de corps (10) insérable dans le fémur d'un animal, la partie de corps comprenant une partie de croissance osseuse (11) formée dans la partie proximale pour favoriser la croissance osseuse et une partie de tige (13) formée dans la partie distale pour faciliter l'insertion dans le fémur, la partie de croissance osseuse (11) comprenant une partie poreuse (111) ayant une pluralité de pores formés dans celle-ci pour favoriser la croissance osseuse,
une pièce d'accouplement (50) à laquelle une tête fémorale artificielle est accouplée, et
une partie de col (30) reliant la partie de corps et la partie d'accouplement, **caractérisé en ce que**
la partie de croissance osseuse (11) est formée pour s'étendre dans la direction distale tout en étant étendue à partir de l'extrémité distale de la partie de col (30) pour former une marche (S),
**que** la partie tige (13) est formée pour s'étendre dans la direction distale tout en étant réduite à partir de l'extrémité distale de la partie de croissance osseuse (11) pour former une marche,
**que** la pièce de croissance osseuse (11) comprend en outre une partie de cadre (113) qui forme une face solide pour protéger la partie poreuse (111), et
**que** la partie de cadre (113) est formée le long du bord de la partie de croissance osseuse (11).

2. Implant fémoral pour animaux selon la revendication 1, dans lequel la partie poreuse et la partie de cadre sont fabriquées par impression 3D.

3. Implant fémoral pour animaux selon la revendication 1, dans lequel la partie poreuse et la partie de cadre, qui sont adjacentes, ont la même hauteur.

4. Implant fémoral pour animaux selon la revendication 1, dans lequel une taille de la partie de col varie en fonction d'une taille de la partie d'accouplement.

5. Implant fémoral pour animaux selon la revendication 4, dans lequel à mesure que la taille de la partie d'accouplement augmente, la taille de la partie de col augmente, et dans lequel à mesure que la taille de la partie d'accouplement diminue, la taille de la partie de col diminue.

6. Implant fémoral pour animaux selon la revendication 1,
dans lequel la pièce de croissance osseuse (11) comprend en outre un trou de verrouillage (115) dans lequel un boulon de verrouillage peut être inséré pour empêcher l'affaissement.

7. Implant fémoral pour animaux selon la revendication 6, dans lequel le trou de verrouillage (115) est formé sur la surface extérieure de la partie de croissance osseuse (11).

8. Implant fémoral pour animaux selon la revendication 7, dans lequel un axe central de trou du trou de verrouillage (115) est perpendiculaire à un axe de tige.

9. Implant fémoral pour animaux selon la revendication 7, dans lequel un axe central de trou du trou de verrouillage (115) n'est pas perpendiculaire à l'axe de tige.
